# EUROPEAN PATENT APPLICATION

(11) **EP 4 289 956 A1**
(43) Date of publication of application: **13.12.2023**
(21) Application number: 22749238.6
(22) Date of filing: 07.02.2022
(51) Int. Cl.: C12N 15/50, C12N 15/11, C12N 15/86, C12N 15/64

(54) **CIS-REPLICON CONSTRUCT**

(30) Priority: 03.02.2021 CN 202110151575; 09.10.2021 CN 202111178173
(71) Applicant: Zhengzhou University, High-Tech Zone Zhengzhou Henan 450001 (CN)
(72) Inventor: ZHANG, Shoutao, Zhengzhou, Henan 450001 (CN); MA, Qiang, Zhengzhou, Henan 450001 (CN); TIAN, Qingnan, Zhengzhou, Henan 450001 (CN); WANG, Tian, Zhengzhou, Henan 450001 (CN); HUANG, Yonghui, Zhengzhou, Henan 450001 (CN); LIU, Guotao, Zhengzhou, Henan 450001 (CN); TIAN, Zhen, Zhengzhou, Henan 450001 (CN)
(74) Representative: Studio Torta S.p.A.
(86) International application number: PCT/CN2022/075384
(87) International publication number: WO 2022/166959

(57) **Abstract**

Provided is a new-type cis-replicon construct for efficiently expressing a target protein, which comprises an RNA polymerase coding unit and a target protein coding unit. The replicon construct can drive the replication of the target protein coding unit and reduce or avoid the replication and expression of the RNA polymerase, thereby effectively improving the expression of the target protein and reducing the expression of the non-target protein.

## Description

This application claims the priority of Chinese Patent Application No. 202110151575.3 filed with the China National Intellectual Property Administration on February 03, 2021, and Chinese Patent Application No. 202111178173.9 filed with the China National Intellectual Property Administration on October 09, 2021.

### FIELD

The present disclosure relates to the technical field of genetic engineering, in particular to a cis-replicon RNA construct for efficiently expressing a target protein.

### BACKGROUND

The introduction of one or more exogenous polypeptides/proteins for the purpose of prevention and treatment of diseases has become more and more widespread in biomedical therapy, and nucleic acid molecules (DNA or mRNA) can be delivered to target cells or organisms through a variety of technical methods. The delivery of mRNA molecules has received increasing attention in recent years, and *in vitro* transcribed (IVT) mRNA has recently come into the spotlight as a potential new drug for delivering genetic information. The use of mRNA has some prominent advantages over that of DNA. First, higher safety: since mRNA is a non-infectious, non-integrating material, there is no potential risk of infection or insertion mutation. Also, mRNA is degraded during normal cellular processes and its *in vivo* half-life can be modulated by using various modification and delivery methods. Moreover, the inherent immunogenicity of mRNA can be downregulated to further improve safety. Second, higher efficiency: various modifications make mRNA more stable and highly translatable, and by *in vivo* administration, mRNA can be prepared into an effective carrier molecule to be quickly taken up and expressed in the cytoplasm. Third, rapid production: the preparation of mRNA has the potential to be fast, cheap, and massively scalable, mainly due to the high yield of *in vitro* transcription reactions, which is important for the pandemic of infectious diseases and the potential to prepare cancer vaccine targeting patient-specific cancer-associated or mutated antigens (Norbert Pardi et al., 2018, Nat Rev Drug Discov., Vol. 17, pp. 261-279).

Alphavirus, a genus of the togavirus family with a positive-strand RNA genome, contains a non-segmented single-stranded RNA genome of 11-12 kb with a type 0 cap (N7mGppp) on the 5' end and a poly(A) tail on the 3' end, which make the alphavirus genome similar to an mRNA that can be translated immediately upon entry into the host cytoplasm and control the production of all viral proteins required to initiate viral replication. Alphavirus includes about 30 members, including Sindbis virus (SINV), Semliki Forest virus (SFV), Venezuelan equine encephalomyelitis virus (VEEV) and Chikungunya virus (CHIKV) etc. (Jennifer L. Hyde et al., Virus Research, 2015, Vol. 206, pp. 99-107). The alphavirus genome has two open reading frames in total, where the first 2/3 part near the 5' end encodes four non-structural proteins (nsP1-nsP4), which is called the non-structural region, and the last 1/3 near the 3' end is called the structural region encoding structural proteins translated by subgenomic mRNA, including capsid protein C, membrane glycoproteins E1, E2 and E3, and 6K protein. The non-structural protein at the 5' end of the alphavirus is first translated during the process of infecting cells to produce the replicase necessary for RNA replication and transcription. Under the action of replicase, the transcription of subgenomic mRNA is initiated, followed by translation, and is cleaved into capsid protein and membrane glycoprotein under the combined effect of viral and host-encoded proteases. These structural proteins are assembled with viral genomic RNA to form nucleocapsid and released by budding. Subgenomic RNA is neither used as a template for RNA synthesis nor packaged into virus particles, and the structural gene can reach a very high copy number during virus replication. Therefore, the subgenomic promoter can be used to initiate the high-efficiency expression of exogenous genes.

The mRNA for *in vivo* delivery can be either a non-amplified mRNA molecule (approximately 2kb) encoding the target protein or a larger, self-amplifying mRNA molecule (> 10,000bp) based on a viral replicon (Piotr S. Kowalski et al., Mol Ther., 2019, Vol. 27, pp. 710-728; Giulietta Maruggi et al., Mol Ther., 2019, Vol. 27, pp. 757-772). The most currently used self-amplifying mRNA (SAM) vaccines are based on the alphavirus genome, in which the gene encoding RNA replicase is intact, but the genes encoding structural proteins are replaced by the gene encoding the target protein. The first-round translation products of viral genomic RNA are assembled into replicase, through which the negative strand (cRNA) complementary to the genome is produced and serves as a template for the synthesis of other mRNA molecules. Due to intracellular replication of the antigen-encoding RNA, the SAM platform enables the production of large quantities of antigens from extremely small doses of vaccines (Norbert Pardi et al., 2018, Nat Rev Drug Discov., Vol. 17, pp. 261-279).

Alphavirus replicons have the following advantages. First, self-amplifying mRNA can increase the expression of exogenous proteins and activate a stronger immune response *in vivo.* This is because the production of exogenous proteins in SAM-transfected cells is based on the principle of viral replication. Under the action of RNA replicase, RNA replicons replicate in large numbers in the cytoplasm and express exogenous genes efficiently, providing an ideal conformation for sustained B cell stimulation and antibody production. In addition, host cells recognize transcribed double-stranded RNA intermediates through a variety of pattern recognition receptors, such as RNA-dependent protein kinase (PKR), Toll-like receptor (TLR) 19, and 2'-5'-oligoadenylate synthase (OAS), which causes local inflammation that provides additional immune stimulation and thus induces higher level of humoral and cellular immune responses (Khromykh A et al., 2000, Curr Opin Mol Ther, Vol. 2, pp. 555- 569 pp.; Annette B. Vogel et al., Mol Ther., 2018, Vol. 26, pp. 446-455). Second, self-amplified mRNA is safe. The alphavirus replicon does not enter the nucleus, but only briefly replicates, transcribes and expresses the target protein in the cytoplasm, and is then eliminated by cell apoptosis under the action of the antiviral signal activated by dsRNA. Therefore, compared with traditional DNA vaccines, it will neither be integrated into the host genome to cause cancer risk, nor existing in the body for a long time (Annette B. Vogel et al., Mol Ther., 2018, Vol. 26, pp. 446-455). Third, it has a wide range of applications. Alphavirus replicons can be efficiently applied to the vaccine research of various pathogens, such as bovine viral diarrhea virus (BVDV), cytomegalovirus (CMV), classical swine fever virus (CSFV), dengue virus (DENV), Ebolavirus, hepatitis B virus (HBV), hepatitis C virus (HCV), hepatitis E virus (HeV), human immunodeficiency virus (HIV), human papillomavirus (HPV), herpes simplex virus (HSV), infectious bursal disease virus (IBDV), influenza virus, infectious salmon anemia virus (ISAV), Japanese encephalitis virus (JEV), Lassa virus, louping-ill virus (LIV), Marburg virus (MBGV), measles virus, Murray Valley encephalitis virus (MVEV), Nipah virus (NiV), Norwalk-like virus (NLV), rabies virus (RV), respiratory syncytial virus (RSV), Rift Valley fever virus (RVFV), severe acute respiratory syndrome coronavirus (SARS-CoV), vaccinia virus, Prion, B. anthracis, B. abortus, C. botulinum, Malaria, M. tuberculosis, P. falciparum, Staphylococcus, etc. (Kenneth Lundstrom, Viruses, 2014, Vol. 6, pp. 2392-2415; Kenneth Lundstrom, Vaccines, 2019, Vol. 7, p. 29; Cuiling Zhang et al., Front Immunol., 2019, Vol. 10, p. 594).

In addition, a safe, effective and controllable gene delivery system is a key factor in gene delivery, currently including viral vectors and non-viral vectors. Non-viral vectors can carry a higher genetic load, have a higher safety profile and induce a lower immune response. Non-viral vectors used to deliver mRNA include polyplexes (PP), lipoplexes (LP), lipid nanoparticles (LNP) and cationic nanoemulsions (CNE). Among them, lipid carriers are the most widely used (S Guan & J Rosenecker, Gene Therapy, 2017, Vol. 24, pp. 133-143; Piotr S. Kowalski et al., Mol Ther., 2019, Vol. 27, pp. 710-728; Kristie Bloom et al., Gene Ther., 2020, pp. 1-13).

Therefore, it is necessary to develop an improved RNA replicon constructs for high-efficiency expression of the target protein, so as to minimize the expression of non-target proteins and greatly amplify the target RNA to increase the expression of the target protein.

### SUMMARY

The purpose of the present disclosure is to provide a cis-replicon RNA construct that efficiently expresses the target protein, so as to solve the above problems in the prior art. The RNA replicon is constructed by modifying the alphavirus genome to drive the high-efficiency replication and expression of the target gene and minimize the expression of non-target proteins, so as to increase the expression of target proteins.

To achieve the above purposes, the present disclosure provides the following schemes:

The present disclosure provides a cis-replicon RNA construct for efficiently expressing a target protein, the construct comprises:
a 5' UTR comprising one or more nucleotide sequences encoding a replicase, wherein the 5' UTR comprises a 5' cap;
a 5' end recognition sequence and a subgenomic promoter that guide the replication of the RNA replicon;
a heterologous nucleotide sequence controlled by the subgenomic promoter;
a multiple cloning site; and
a 3' UTR that ensures the stability of RNA and the effective replication of the target gene, wherein the 3' UTR comprises a 3' end recognition sequence and a 3' polyA that guide the replication of the RNA replicon.

In some embodiments, the present disclosure provides a cis-replicon RNA construct for efficiently expressing a target protein, wherein the construct, in the 5'-3' direction, comprises
(1) a 5' UTR sequence comprising a 5' cap structure;
(2) one or more nucleotide sequences encoding a replicase;
(3) a 5' end recognition sequence and a subgenomic promoter (SGP) that guide the replication of the RNA replicon;
(4) a multiple cloning site;
(5) a target gene or heterologous nucleotide sequence controlled by the subgenomic promoter;
(6) a 3' end recognition sequence that guides the replication of the RNA replicon; and
(7) a 3' UTR and 3' poly(A) that ensure the stability of RNA and the effective replication of the target gene.

Preferably, the replicase is derived from alphavirus.

Preferably, the alphavirus is Semliki Forest virus, Venezuelan equine encephalitis virus, Sindbis virus, or Chikungunya virus.

In some embodiments, the 5' end recognition sequence and the 3' end recognition sequence guide the replication of the RNA replicon in the presence of the replicase; and the 5' end recognition sequence and the 3' end recognition sequence comprise CSE1 and CSE2.

In some embodiments, the 5' end recognition sequence and the 3' end recognition sequence of the alphavirus are preferably of the replicase origin, and the 5' UTR is a eukaryotic 5' UTR;
the 5' cap structure is a natural 5'-cap or a 5'-cap analog;
the 3' UTR is the 3' UTR of a viral mRNA.

In some embodiments, the nucleotide sequence encoding the replicase comprises an open reading frame, and the open reading frame encoding the replicase comprises a coding region for a nonstructural protein required for RNA replication.

In some embodiments, the RNA construct used to express an alphavirus replicase and/or the RNA replicon does not comprise an open reading frame encoding a complete alphavirus structural protein.

In some embodiments, the heterologous nucleotide sequence encodes a target protein, and the expression of the open reading frame encoding the target protein is controlled by a subgenomic promoter.

In some embodiments, the open reading frame encoding the target protein is non-native to the alphavirus from which the replicase is derived;
the subgenomic promoter is native to the alphavirus from which the replicase is derived.

In some embodiments, the present disclosure provides a recombinant viral vector, plasmid DNA, linear DNA, in vitro transcribed RNA or extracted intracellular transcribed RNA comprising the cis-replicon construct.

The present disclosure also provides a DNA recombination vector comprising the cis-replicon RNA construct, wherein the recombination vector cannot drive the replication of replicase, and efficiently drives the replication and expression of the target gene.

The present disclosure also provides the nucleotide sequence encoding the cis-replicon RNA construct.

The present disclosure also provides a method for producing a protein using an organism, comprising obtaining the construct or the recombinant vector for expressing the target protein, and transfecting the construct or the recombinant vector into the organism.

Further, the organism includes vertebrates and cells.

The present disclosure also provides the following technical schemes:

In a first aspect, the present disclosure provides an expression construct of cis-replicon, comprising an RNA polymerase coding unit and a target protein coding unit, wherein
the RNA polymerase coding unit comprises a nucleic acid fragment encoding an RNA polymerase, and the target protein coding unit comprises an insertion site (such as a multiple cloning site) for a target protein coding fragment and/or a nucleic acid fragment encoding the target protein; and
the construct is capable of expressing the RNA polymerase and replicating itself dependent on the RNA polymerase in a target cell to provide a template for expressing the target protein; preferably, the replication of the target protein coding unit is more efficiently than the replication of the RNA polymerase coding unit; and more preferably, only the target protein coding unit is replicated.

In some embodiments, the expression construct is DNA or RNA, preferably RNA, including single-stranded RNA or double-stranded RNA, such as single-stranded RNA, particularly positive strand (+) single-stranded RNA.

In some embodiments, the expression construct is linear or circular;
particularly, the expression construct is a linear RNA, wherein a 5' cap structure or an IRES sequence is provided in the 5' end of the RNA to drive translation of downstream coding sequence; or the construct is a circular RNA, wherein an IRES sequence is provided upstream of the RNA polymerase coding unit to drive translation of the RNA polymerase coding unit.

In some embodiments, the construct comprises replication initiating elements located both upstream and downstream of the target protein coding unit, wherein the replication initiating elements dependent on the RNA polymerase for the replication of the target protein coding unit; wherein the RNA polymerase coding unit is preferably not located between the upstream replication initiating element and downstream replication initiating element.

In some embodiments, the upstream replication initiating element comprises a CSE1 and/or CSE3 sequence, preferably CSE1 and CSE3 sequences, and more preferably CSE1, CSE2 and CSE3 sequences; and/or the downstream replication initiating element comprises a CSE4 sequence.

In some embodiments, the target protein coding unit is located downstream or upstream of the RNA polymerase coding unit.

In some embodiments, the target protein coding unit is located upstream of the RNA polymerase coding unit, and the target protein and the RNA polymerase are expressed as a fusion protein with a linker between the target protein and the RNA polymerase, wherein the linker comprises a fragment encoding a protease recognition site, and the RNA polymerase is released after the fusion protein is recognized and cleaved by a protease; and preferably, the protease recognition site is a 2A peptide sequence. The fragment encoding a protease recognition site may be located upstream or downstream of the replication initiating element (e.g., CSE4).

In some embodiments, the target protein coding unit is located upstream of the RNA polymerase coding unit, and an IRES sequence is located between the target protein coding unit and the RNA polymerase coding unit to drive translation of the RNA polymerase coding unit. The IRES sequence may be located upstream or downstream of the replication initiating element (e.g., CSE4).

In some embodiments, the target protein coding unit is located upstream of the RNA polymerase coding unit, and a self-cleaving HDV-like ribozyme is located between the target protein coding unit and the RNA polymerase coding unit. The self-cleaving HDV-like ribozyme may be located upstream or downstream of the replication initiating element (e.g., CSE4).

In some embodiments, the target protein coding unit is located downstream of the RNA polymerase coding unit, wherein the construct, in the 5'-3' direction, comprises
a 5' cap structure or an IRES sequence to drive translation of downstream coding sequence, the RNA polymerase coding unit, the upstream replication initiating element, the target protein coding unit and the downstream replication initiating element.

In some embodiments, the expression construct, in the 5'-3' direction, comprises
(1) a 5' UTR sequence comprising a 5' cap structure;
(2) one or more nucleotide sequences encoding the RNA polymerase;
(3) a 5' end recognition sequence and a subgenomic promoter (SGP) that guide the replication of the RNA replicon;
(4) a multiple cloning site and/or a target gene or heterologous nucleotide sequence controlled by the subgenomic promoter;
(5) a 3' end recognition sequence that guides the replication of the RNA replicon; and
(6) a 3' UTR and 3' poly(A) that ensure the stability of RNA and the effective replication of the target gene.

In some embodiments, a subgenomic promoter (SGP) is located upstream of the target protein coding unit, wherein the subgenomic promoter controls the target gene or heterologous nucleotide sequence.

In some embodiments, the expression construct is DNA, wherein a subgenomic promoter (SGP) is located upstream of the RNA polymerase coding unit.

In some embodiments, the RNA polymerase is a viral replicase, particularly a replicase of an alphavirus,
preferably, the alphavirus is selected from the group consisting of Semliki Forest virus, Barmah Forest virus, Chikungunya virus, O'nyong'nyong virus, Ross River virus, Bebaru virus, Getah virus, Sagiyama virus, Mayaro virus, Una virus, Venezuelan equine encephalitis complex or Venezuelan equine encephalitis virus, Cabassou virus, Everglades virus, Mucambo virus, Tonate virus, Pixuna virus, Mosso das pedras virus, Rio Negro virus, Western equine encephalitis complex or Western equine encephalitis virus, Sindbis virus, Aura virus, Whataroa virus, Highlands J virus, Fort Morgan virus, Buggy Creek virus, Eastern equine encephalitis virus, Middelburg virus, Trocara virus, Kyzylagach virus, Ndumu virus, Babanki virus, Norwegian salmonid alphavirus, Salmon pancreatic disease virus, sleeping disease virus and Southern elephant seal virus;
more preferably, the alphavirus is selected from the group consisting of Semliki Forest virus, Venezuelan equine encephalitis virus, Sindbis virus and Chikungunya virus.

In some embodiments, the 5' cap structure is a natural 5' cap or a 5' cap analog.

In some embodiments, the construct comprises a 5' UTR derived from the 5' UTR of any gene or a mutant thereof, preferably a 5' UTR derived from the same virus as which the RNA polymerase derived from.

In some embodiments, the replication initiating element is capable of being recognized by the RNA polymerase and guiding the replication of the RNA polymerase,
preferably, the replication initiating element and the RNA polymerase are derived from the same virus or from different viruses, more preferably from the same virus.

In some embodiments, the construct comprises a 3' UTR derived from the 3' UTR of any gene or a mutant thereof, preferably a 3' UTR derived from the same virus as which the RNA polymerase derived from.

In some embodiments, the construct comprises a 3' poly(A).

In some embodiments, the subgenomic promoter is derived from the promoter of a viral structural protein, and preferably, the subgenomic promoter and the RNA polymerase are derived from the same virus.

In a second aspect, the present disclosure provides a vector comprising or encoding the expression construct of the first aspect.

In a third aspect, the present disclosure provides a vaccine composition comprising the construct of the first aspect or the vector of the second aspect, wherein the target protein is an antigen capable of eliciting a protective immune response, such as an antigen derived from humans, animals, plants, viruses, bacteria and/or parasites.

In the fourth aspect, the present disclosure provides a pharmaceutical composition, which comprises the construct of the first aspect or the vector of the second aspect, wherein the target protein is a therapeutic protein.

In a fifth aspect, the present disclosure provides a method for expressing a target protein, which comprises introducing the construct of the first aspect into a target cell, wherein the target cell expresses the target protein from the construct.

In a sixth aspect, the present disclosure provides a method of immunization comprising inoculating a subject in need thereof with the construct of the first aspect.

The present disclosure discloses the following technical effects:

The cis-replicon RNA construct disclosed by the present disclosure is constructed by modifying the various elements of the alphavirus genome. The constructed replicon cannot drive the replication of replicase, but can only drive the efficient replication and expression of the target gene, which can minimize the expression of the target protein and increase the expression of the target protein by greatly amplifying the target RNA. The present disclosure does not need to cultivate the alphavirus, and can realize the high-efficiency expression of the target protein in cells or organisms, making it suitable for effectively and safely producing target proteins such as therapeutic proteins or antigenic proteins in organisms, which also provides the basis and method for medical application.

### BRIEF DESCRIPTION OF DRAWINGS

In order to more clearly illustrate the technical solutions in the embodiments of the present disclosure or the prior art, the accompanying drawings used in the embodiments will be briefly introduced below. Obviously, the accompanying drawings in the following description are only some embodiments of the present disclosure. For those of ordinary skill in the art, other drawings can also be obtained based on these drawings without any creative effort.

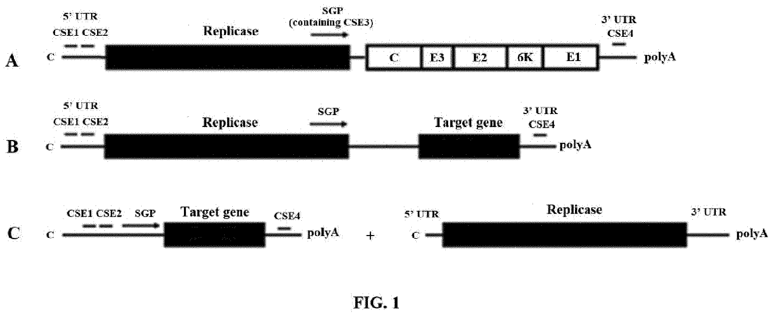

FIG. 1 shows the structures of the replicons in the prior art. FIG. 1A shows the genome structure of the alphavirus. The genome of the alphavirus is a single-stranded (+) strand RNA encoding two open reading frames (ORFs). The first 2/3 part near the 5' end encodes 4 nonstructural proteins (nsP1-nsP4), which are translated and processed as replicase; and the last 1/3 near the 3' end is called the structural region, the transcription of which is under the control of the subgenomic promoter (SGP) and encodes structural proteins such as capsid protein C, membrane glycoproteins E1, E2 and E3, and 6K protein. FIG. 1B shows the structure of a traditional cis-replicon, in which the region encoding the structural protein is replaced by the coding region of the target gene. FIG. 1C shows a structure of a replicon (also referred to herein as a trans-replicon), in which the target gene and the replicase are located in two separate constructs. Abbreviations: C: 5' cap; SGP: subgenomic promoter; CSE: conserved sequence element.

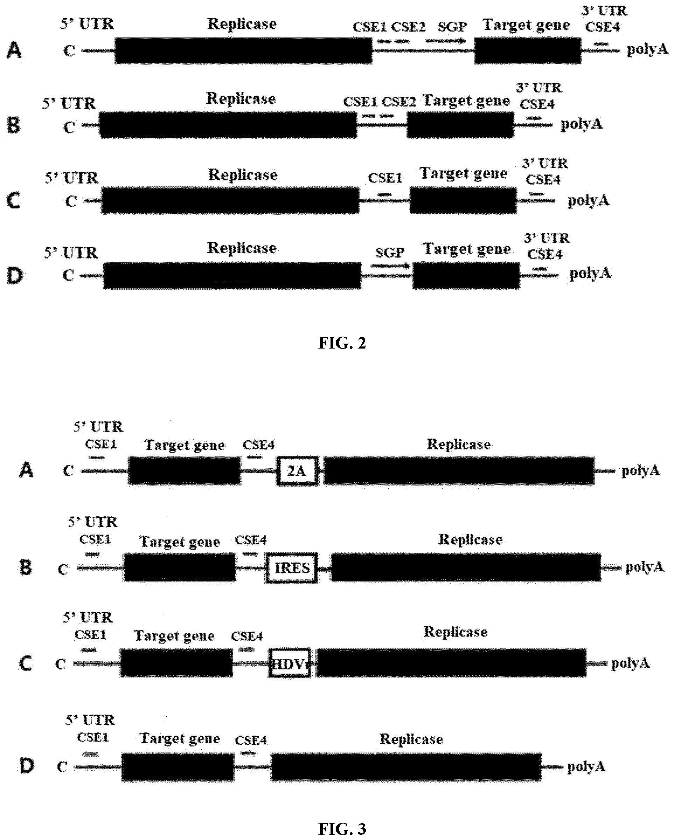

FIG. 2 shows the structures of the replicons in some embodiments of the present disclosure, in which the replicase coding unit is located upstream of the target gene coding unit. FIG. 2A shows that the upstream replication initiating element comprises CSE1, CSE2 and SGP, and the downstream replication initiating element comprises CSE4; FIG. 2B shows the upstream replication initiating element comprising CSE1 and CSE2 and the downstream replication initiating element comprising CSE4; FIG. 2C shows the upstream replication initiating element comprising CSE1 and the downstream replication initiating element comprising CSE4; FIG. 2D shows the upstream replication initiating element comprising SGP and the downstream replication initiating element comprising CSE4.
FIG. 3 shows the structures of the cis-replicons in some embodiments of the present disclosure, in which the replicase coding unit is located downstream of the target gene coding unit. FIG. 3A shows that there is a 2A peptide sequence between the replicase coding unit and the target gene coding unit; FIG. 3B shows that there is an IRES sequence between the replicase coding unit and the target gene coding unit; FIG. 3C shows that there is an HDV-like ribozyme (HDVr) sequence between the replicase coding unit and the target gene coding unit; FIG. 3D shows that there is no self-cleavage sequence or IRES sequence between the replicase coding unit and the target gene coding unit.

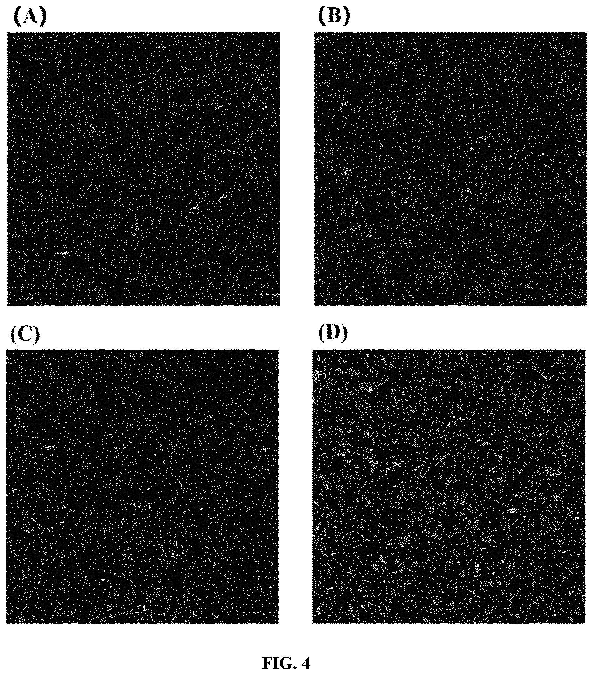

FIG. 4 shows the fluorescence images of cells in which the exogenous protein (fluorescent protein EGFP) is expressed by different replicons: (A) fluorescence signals of EGFP expression by the traditional plasmid, (B) by the traditional cis-replicon with the structure shown in FIG. 1B, (C) by the trans-replicon with the structure shown in FIG. 1C, and (D) by the cis-repliconwith the structure shown in FIG. 2A.

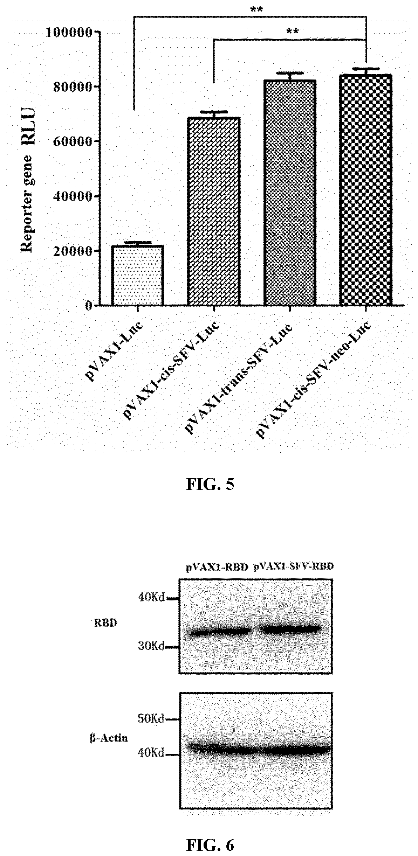

FIG. 5 shows the signal detection results of the reporter gene (Luc as the reporter gene) which are expressed by different replicons: the traditional plasmid, the cis-replicon with the structure shown in FIG. 1B, the trans-replicon with the structure shown in FIG. 1C, and the cis-replicon of the present disclosure with the structure shown in FIG. 2A, respectively.
FIG. 6 shows the WB detection results of exogenous protein (new coronavirus RBD protein) which is expressed by different replicons: the traditional plasmid and the cis-replicon of the present disclosure with the structure shown in FIG. 2A.

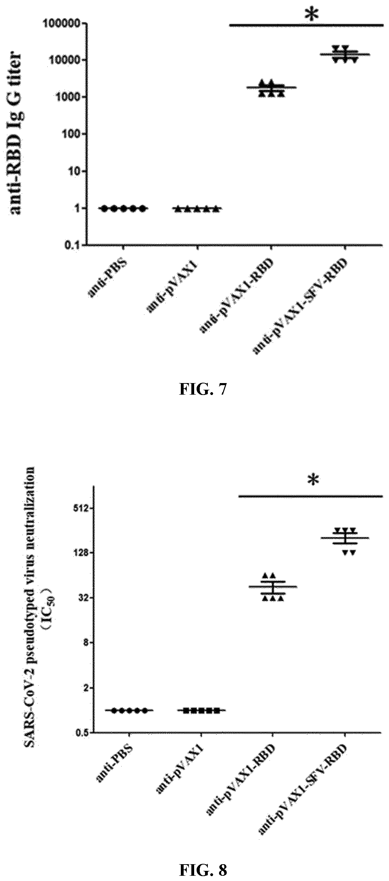

FIG. 7 shows the IgG titer of anti-SARS-CoV-2-RBD in the serum of immunized mice measured by ELISA.
FIG. 8 shows the neutralization titer (IC₅₀) of the anti-SARS-CoV-2-RBD antibody in the serum of immunized mice measured in the pseudovirus neutralization assay.

### DETAILED DESCRIPTION

### Terms

The term "replicon" in the present disclosure refers to a unit capable of independent replication, which comprises one or more elements required for replication and may be DNA or RNA. "Cis-replicon" means that the elements required for replication are located on the same polynucleotide chain, which can be linear or circular. "Trans-replicon" means that the elements required for replication are located on different polynucleotide chains.

The term "target protein coding unit" in the present disclosure refers to a polynucleotide fragment comprising the target protein coding sequence and/or an insertion site (such as a multiple cloning site) into which a target protein coding sequence is to be inserted. The term "RNA polymerase coding unit" refers to a polynucleotide fragment comprising an RNA polymerase coding sequence.

The term "replication" in the present disclosure refers to the generation of a polynucleotide chain identical to the full length or fragment of a template polynucleotide chain by using RNA polymerase. In some embodiments, the replication involves replication of a sequence between an upstream replication initiating element and a downstream replication initiating element, resulting in multiple identical copies.

The term "replication initiating element" in the present disclosure refers to a polynucleotide fragment that can be recognized by RNA polymerase (e.g., viral replicase) and initiates replication.

The term "upstream/downstream of ..." described herein should be understood in the 5'-3' direction for a specific polynucleotide sequence to define upstream and downstream.

### Alphavirus

In the present disclosure, the term "alphavirus" should be broadly understood and includes any virus particle having the characteristics of an alphavirus. Alphaviruses have characteristics including the presence of (+) strand RNA that encodes genetic information suitable for replication in host cells, including replicase, etc. Further characterization of alphavirus is described in, for example, Strauss & Strauss, Microbiol. Rev., 1994, Vol. 58, pp. 491-562; Gould et al., 2010, Antiviral Res., Vol. 87, pp. 111-124; Jonathan Rupp et al., 2015, J. Gen. Virology, Vol. 96, pp. 2483-2500; Kathryn Carpentier et al., Curr Opin Virol, 2018, Vol. 28, pp. 53-60; Luis Carrasco et al., Viruses, 2018, Vol. 10, p. 70; Rebecca Brown et al., Viruses, 2018, Vol. 10, p. 89; Autumn Holmes et al., PLoS Pathog, 2020, Vol. 16, e1008876, et al. The term "alphavirus" includes alphaviruses found in nature and any variants or derivatives thereof.

Alphaviruses found in nature are preferably selected from the group consisting of Semliki Forest virus complex or Semliki Forest virus, Barmah Forest virus, Chikungunya virus, O'nyong'nyong virus, Ross River virus, Bebaru virus, Getah virus, Sagiyama virus, Mayaro virus, Una virus, Venezuelan equine encephalitis complex or Venezuelan equine encephalitis virus, Cabassou virus, Everglades virus, Mucambo virus, Tonate virus, Pixuna virus, Mosso das pedras virus, Rio Negro virus, Western equine encephalitis complex or Western equine encephalitis virus, Sindbis virus, Aura virus, Whataroa virus, Highlands J virus, Fort Morgan virus, Buggy Creek virus, Eastern equine encephalitis virus, Middelburg virus, Trocara virus, Kyzylagach virus, Ndumu virus, Babanki virus, Norwegian salmonid alphavirus, Salmon pancreatic disease virus, sleeping disease virus and Southern elephant seal virus, etc.

More preferably, the alphavirus is selected from the group consisting of Semliki Forest virus complex or its virus types described above including Semliki Forest virus, Western Equine Encephalitis complex or its virus types described above including Sindbis virus, Venezuelan equine encephalitis complex or its virus types described above including Venezuelan equine encephalitis virus.

In some embodiments of the present disclosure, the alphavirus is not an alphavirus found in nature, but an alphavirus modified by genetic engineering. Generally, alphaviruses not found in nature are variants or derivatives of alphaviruses found in nature, which differ from alphaviruses found in nature in at least one mutation in the nucleotide sequence (i.e., genomic RNA). Mutations in the nucleotide sequence may be selected from insertions, substitutions or deletions of one or more nucleotides compared to alphaviruses found in nature. A mutation in a nucleotide sequence may or may not be related to a mutation in the polypeptide or protein encoded by the nucleotide sequence. For example, Erkuden Casales et al. used puromycin to screen the double-mutant replicase R649H/P718T of SFV expression vector, which expressed a level of β-galactose similar to that of the parental vector, and could maintain a stable high expression level during at least 10 passages (Erkuden Casales et al., Virology, 2008, Vol. 376, pp. 242-251). Toey Nivitchanyong et al. performed point mutation on the non-structural protein (nsP) P726S to construct two non-cytopathic and replication-active virus strains of Sindbis virus, TE and 633, which can sustainably express heterologous genes after infecting baby hamster kidney (BHK) cells and Chinese hamster ovary (CHO) cells (Toey Nivitchanyong et al., Virus Res., 2009, Vol. 141, pp. 1-12).

### Characterization of replicase constructs

The genome of alphavirus has 2 open reading frames, and the first 2/3 part near the 5' end encodes four non-structural proteins (nsP1-4) called non-structural regions. In the process of infecting cells, the non-structural proteins at the 5' end are first translated into a large polyprotein, which produces the replicase complex required for RNA replication and transcription, and uses genomic RNA as a template to synthesize the negative strand. This process occurs within 3-4 hours after cell infection, and then only positive strand RNA will be synthesized.

The cis-replicon RNA construct of the present disclosure contains an open reading frame (ORF) encoding alphavirus replicase. In the present disclosure, "nonstructural protein" is any one or more individual nonstructural proteins (nsP1, nsP2, nsP3, nsP4) derived from alphavirus, or a polyprotein comprising the polypeptide sequence of more than one nonstructural protein derived from alphavirus, such as nsP1234. In the examples, "nonstructural protein" refers to nsP123.

nsP1 is a guanine-7-methyltransferase (also known as a viral cap enzyme) with methyltransferase and guanylyltransferase activities, as well as a membrane anchoring function for the replication complex. Its methyltransferase domain catalyzes the addition of the methyl group of S-adenosylmethionine to the GTP molecule to form a covalent m7GMP-nsP1 intermediate product. After nsP2 removes the 5'-γ-phosphate on the positive-strand RNA molecule, the m7GMP part is transferred to the RNA molecule by the guanosyltransferase activity of nsP1, forming a 0-type cap structure, m7GpppA (Leevi Kääriäinen & Tero Ahola, 2002, Vol. 71, pp. 187-222; Maija K. Pietilä et al., 2017, Vol. 234, pp. 44-57; Autumn T. LaPointe et al., mBio., 2018, Vol. 9, pp. e02342-18).

nsP2 is the largest replicase protein and is an RNA-binding protein with nucleoside-triphosphatase (NTPase), RNA helicase and protease activities, which can open RNA double strands and assist nsP1 protein to complete the capping process. The protease activity of nsP2 also plays an important role in the decomposition of replicase into non-structural proteins (Ellen G. Strauss et al., Virology, 1992, Vol. 191, pp. 932-940; M Gomez de Cedrón et al., FEBS Lett, 1999, Vol. 448, pp. 19-22; Leevi Kääriäinen & Tero Ahola, 2002, Vol. 71, pp. 187-222; Maija K. Pietilä et al., 2017, Vol. 234, pp. 44-57).

nsP3 is a phosphorylated protein consisting of three domains: N-terminal conserved domain with phosphatase activity and nucleic acid binding ability, alphavirus unique domain (AUD) and C-terminal hypervariable domain (HVD) (Farhana Abu Bakar et al., Viruses., 2018, Vol. 10, p. 71). The function of nsP3 is poorly understood and is known as one of the more mysterious nsPs. In recent years, nsP3 has been found to interact with several host proteins, including stress granule-associated proteins, DEAD-box family proteins, heat shock proteins, and kinases, and may regulate protein poly/mono-ADP ribosylation (Tyler Lark et al., Front Microbiol, 2017, Vol. 8, p. 2652; Maija K. Pietilä et al., 2017, Vol. 234, pp. 44-57; Benjamin Götte et al., Viruses, 2018, Vol. 10, p. 105). Furthermore, nsP3 may also be involved in neurovirulence with Old World alphaviruses such as SFV (Benjamin Götte et al., Viruses, 2018, Vol. 10, p. 105).

nsP4 contains an RNA-dependent RNA polymerase (RdRp) domain at the C-terminus and is the most conserved protein in alphaviruses. As the main catalytic core of the alphavirus replication complex, nsP4 forms a replication complex (RC) together with host proteins and other nsPs (such as nsP123+nsP4), and synthesizes (-) strand RNA using the viral (+) strand as a template. After nsP123 is cleaved into nsP1, nsP2 and nsP3, it can form a tetramer with nsP4, which no longer has the function of synthesizing negative-strand RNA, but it can use negative-strand RNA as a template to participate in the replication of genomic RNA and the transcription of 26S subgenomic RNA. Purified nsP4 also has terminal adenylyltransferase activity, which may generate a poly(A) tail at the 3' end of the genome (Maija K. Pietilä et al., 2017, Vol. 234, pp. 44-57; Farhana Abu Bakar et al., Viruses., 2018, Vol. 10, p. 71).

The origin of the replicase is not limited to any particular alphavirus. In a preferred embodiment, the alphavirus replicase is derived from Semliki Forest virus (SFV), including naturally occurring Semliki Forest virus or variants thereof. In an alternative preferred embodiment, the replicase is derived from Sindbis virus (SINV), including naturally occurring Sindbis virus or variants thereof. In an alternative preferred embodiment, the replicase is derived from Venezuelan Equine Encephalitis virus (VEEV), including naturally occurring VEEV or variants thereof.

### UTR

The term "untranslated region" or "UTR" refers to a region that is transcribed but not translated into an amino acid sequence, or the corresponding region in an RNA molecule such as mRNA. The 5' UTR and 3' UTR of alphavirus contain distinct core promoter elements for negative- and positive-strand RNA synthesis. The alphavirus capped 5' UTR contains a promoter element, translational regulatory sequences, and structures of innate immune defense (Jennifer L. Hyde et al., Virus Research, 2015, Vol. 206, pp. 99-107).

The alphavirus 5' UTR contains core promoter elements that act together with other cis-acting elements downstream and within the 3' UTR to regulate the synthesis of positive- and negative-strands (Kulasegaran Shylini et al., 2009, J Virol., pp. 8327-8339). The adenylated 3' UTR of alphaviruses contains highly conserved viral replication sequence elements, elements that determine cell tropism, and conserved binding regions that affect viral RNA stability (Jennifer L. Hyde et al., Virus Res, 2015, pp. 99-107). The common conserved sequence (CSE) of 19 nucleotides at the end of poly(A) is the promoter for the synthesis of the negative-strand (Kuhn et al., J Virol, 1990, pages 1465-1476), and poly(A) tail and CSE together support the synthesis and efficient translation of the negative-strand, which is a necessary step for virus replication.

Preferably, the cis-RNA replicon of the present disclosure comprises a 5' UTR of non-viral origin, particularly non-alphavirus origin such as human β-globin 5' UTR, which can improve translation efficiency while allowing replicase not to amplify the RNA encoding the replicase region. In one embodiment, the RNA comprises a 5' UTR derived from a eukaryote. A 5' UTR according to the present disclosure may comprise any combination of more than one nucleic acid sequence, optionally separated by a linker.

Preferably, the cis-RNA replicon of the present disclosure comprises a 3' UTR of an alphavirus, and the 3' UTR is active, which can ensure the stability of RNA and the effective replication of the target gene.

### Poly(A) sequence

The cis-RNA construct of the present disclosure comprises a 3' poly(A) sequence. During alphavirus replication, the replication complex and the poly(A) tail interact via the poly(A) binding protein (PABP), so the poly(A) tail requires at least 11-12 residues to effectively generate negative-strand RNA. The synthesis of the negative-strand replicates with comparable efficiency on RNA templates with poly(A) tails containing 25 or 34 adenylate residues; however, the efficiency of negative-strand synthesis decreases significantly (>70%) when the tail size is reduced to 20 residues, and decreases further when the tail size is reduced to 10 residues. Deletion of the 3' poly(A) tail severely inhibits the generation of negative-strand RNA. The negative-strand RNA synthesis of a template RNA without a 3' poly(A) tail is only 4% of that of a template RNA containing 25 3'-terminal adenine residues (Hardy and Rice, 2005, J Virol, Vol. 79 , pp. 4630-4639).

In addition, the poly(A) sequence also affects the function of RNA stability and protein translation in transfected eukaryotic cells. A 3'poly(A) sequence of approximately 120 A nucleotides has been reported to have beneficial effects on RNA levels in transfected eukaryotic cells as well as protein levels translated from the open reading frame upstream of the 3'poly(A) sequence (Nadine Bangel-Ruland, J Gene Med, 2013, Vol. 15, pp. 414-426). Furthermore, the use of segmented poly(A), such as poly(A) 2 × 60_G or poly(A) 2 × 60_T, can significantly reduce plasmid recombination in E. coli without any negative effect on mRNA half-life and protein expression. This poly(A) tail is characterized in that it consists of at least two A-containing elements, each defined as a nucleotide sequence consisting of 40-60 adenine nucleotides, separated by spacer elements of varying length. Compared to conventional homogeneous poly(A) tails, segmented poly(A) tails have a higher potential in terms of recombinant plasmid and resulting mRNA properties (half-life and translation efficiency) (Zeljka Trepotec et al.; RNA, 2019 25, pp. 507-518).

According to the present disclosure, in an embodiment, the 3'poly(A) sequence comprises or consists essentially of or consists of at least 11, preferably at least 25, preferably at least 34, preferably at least 69, preferably at least 100, and preferably at most 500, preferably at most 400, preferably at most 300, preferably at most 200, especially at most 150 A nucleotides, especially about 120 A nucleotides. In the present disclosure, at least 50%, preferably at least 75%, of the poly(A) sequence are A nucleotides, but the remaining nucleotides are allowed to be nucleotides other than A nucleotides, such as U nucleotides (uridylic acid), G nucleotides (guanylic acid), C nucleotides (cytidylic acid), in order to form a segmented poly(A) tail.

### Codon optimization

The use of 3-letter codons for mRNA means that 64 possible codons encode 20 amino acids and a translation stop signal. The degeneracy of the genetic code means that in several triplet genetic codes encoding the same amino acid, the first and second bases are mostly the same, but the third base is different. There are multiple codons for the same amino acid, and since tRNAs randomly recognize codons within the ribosomal A site, codons can be defined as optimal based on the efficiency of selecting a suitable homologous tRNA from the cytoplasmic pool of tRNAs. Codon frequencies used vary across the biological world, and codon bias correlates with tRNA levels in Escherichia coli, Saccharomyces cerevisiae, C. elegans, Drosophila, and eukaryotes (Gavin Hanson et al., Nat Rev Mol Cell Biol., 2018, Vol. 19, pp. 20-30). The main strategy of codon optimization is to increase translation elongation by overcoming constraints associated with species-specific differences in codon utilization and tRNA abundance. There are several methods for redesigning gene coding, such as using biased codons, avoiding rare codons with low utilization rates, simplifying the secondary structure of mRNA after gene transcription, removing motifs that are not conducive to high-efficiency expression, adding motifs that are conducive to high-efficiency expression, and adjusting GC content, etc. In addition to codon usage bias, the combination efficiency of codons also needs to be considered. In most cases, the effect of using only high-frequency codons will not be the best, and the combination of high-frequency and sub-high-frequency codons is considered to be better (Vincent P Mauro et al., BioDrugs., 2018, p. 32, pp. 69-81). In some embodiments of the present disclosure, the codons of the open reading frame contained by the RNA molecule may be different from the corresponding codons from which the open reading frame originated, and will be optimized according to different species, referred to as "codon optimization". In summary, codon optimization can be performed on the open reading frames encoding the replicase and the expressed exogenous protein of the present disclosure, respectively.

### Conserved sequence element (CSE)

In the present disclosure, the term "conserved sequence element" or "CSE" refers to a nucleotide sequence found in alphavirus RNA. These sequence elements are termed "conserved" because orthologs exist in the genomes of different alphaviruses, and orthologous CSEs of different alphaviruses preferably share a high percentage of sequence and/or similar secondary or tertiary structure. Four conserved sequence elements (CSEs) are found in the alphavirus genome, which perform specific functions during viral RNA replication. In one embodiment, the replicon according to the present disclosure comprises one or more conserved sequence elements (CSEs) (Strauss & Strauss, Microbiol. Rev., 1994, Vol. 58, pp. 491-562). For example, in Sindbis virus, the first 44 nucleotides of the positive strand 5' UTR form a conserved stem-loop structure, and its complementary sequence is located at the 3' end of the negative strand, which is a promoter for synthesizing positive-strand RNA with negative-strand RNA as a template. CSE2 is a conserved stem-loop structure formed by the front 51 nucleotides of the nsP1 protein coding sequence, and is a co-promoter for synthesizing negative-strand RNA with positive-strand RNA as a template. CSE3 is 24 nucleotides at the end of the nsP4 protein and is a promoter for transcription of subgenomic mRNA. CSE4 is 19 nucleotides at the 3' end of RNA and is a promoter for synthesizing negative-strand RNA with positive-strand RNA as a template (Hardy and Rice, J. Virol., 2005, Vol. 79, pp. 4630-4639).

In an embodiment, the alphavirus 5' replication recognition sequence and the alphavirus 3' replication recognition sequence are capable of guiding the replication of the RNA replicon in the presence of replicase. Thus, when present alone or preferably together, these recognition sequences guide the replication of the RNA replicon in the presence of replicase. Without wishing to be bound by any particular theory, it is understood that alphavirus conserved sequence elements are recognition sequences that guide RNA replicon replication in the presence of replicase. In an embodiment, the replicon recognition sequences are generally referred to as CSE 1 and 2. Furthermore, in an alternative embodiment, the replicase and conserved sequence elements may be from different alphaviruses.

CSE-1, CSE-2, CSE-3 and CSE-4 described herein include variants thereof that retain their ability to initiate replication.

### Subgenomic promoter (SGP)

In the present disclosure, the term "subgenomic promoter" or "SGP" refers to a nucleic acid sequence 5' upstream of a nucleic acid sequence (e.g., a coding sequence), which controls the transcription of the nucleic acid sequence by providing a recognition and binding site for replicase. In a particular embodiment, the RNA replicon according to the present disclosure comprises a subgenomic promoter. The SGP described herein includes variants thereof that retain their ability to initiate transcription of downstream genes.

Preferably, the replicase encoded by the present replicon is an alphavirus replicase capable of recognizing a subgenomic promoter.

Preferably, the subgenomic promoter according to the present disclosure is the promoter of the alphavirus structural protein, comprising highly conserved CSE3 in the alphavirus, that is, at least 24 nucleotides at the end of the non-structural protein nsP4 (taking Sindbis virus as an example), which can effectively initiate the transcription of subgenomic RNA. In some embodiments, the SGP is the same as or overlaps with or comprises CSE3.

### Heterologous nucleic acid sequence

In one embodiment, the cis-replicon RNA comprises at least one nucleic acid sequence not derived from an alphavirus. According to the present disclosure, the term "heterologous" or "exogenous" refers to the situation where the nucleic acid sequence is not homologous to the alphavirus genome nucleic acid sequence. Preferably, the heterologous nucleic acid sequence is under the control of an alphavirus subgenomic promoter. More preferably, the heterologous nucleic acid sequence is located downstream of a subgenomic promoter. The alphavirus subgenomic promoter is very efficient and thus suitable for high levels of heterologous gene expression (Kenneth Lundstrom, Molecules, 2018, Vol. 23, p. 3310).

### Target protein

The "target protein" referred to herein is the protein encoded by the target gene coding unit. In one embodiment, the RNA replicon according to the present disclosure comprises an open reading frame encoding a target peptide or target protein. Preferably, the target protein is encoded by a heterologous nucleic acid sequence. Preferably, the target gene is under the control of a subgenomic promoter. More preferably, the target gene is located downstream of a subgenomic promoter.

The term "internal ribosome entry site" (IRES) in the present disclosure refers to a sequence that can recruit ribosomes to initiate translation of downstream genes, and can mediate the internal initiation of translation when present between two coding sequences.

The replicon provided by the present disclosure may encode single or multiple proteins. Multiple proteins can be encoded as fusion expressed proteins or separately expressed proteins. For separately expressed proteins, one or more of these proteins can be provided with an upstream IRES, or the ORFs of multiple proteins can be constructed in the replicon while keeping each ORF under the control of a subgenomic promoter. In addition, an autocatalytic protease such as foot-and-mouth disease virus 2A protein can also be added to these proteins for separation (Strauss & Strauss, Microbiol. Rev., 1994, volume 58, pp. 491-562).

The term "2A peptide" or "2A self-cleaving peptide" in the present disclosure refers to a fragment of about 18-22 amino acids, which can induce self-cleavage of recombinant proteins containing 2A peptide in cells. In some embodiments of the present disclosure, the nucleic acid fragment encoding the 2A peptide is present between the target gene coding unit and the RNA polymerase coding unit.

The term "HDV-like ribozyme" or "Hepatitis Delta Virus (HDV) ribozyme (HDVr)" in the present disclosure refers to a class of ribozymes with self-cleavage function. The HDV ribozyme catalyzes the cleavage of the phosphodiester bond between the substrate nucleotide or oligonucleotide and the 5'-hydroxyl of the ribozyme, thereby breaking the host polynucleotide strand from the cleavage site. In some embodiments of the present disclosure, the HDV-like ribozyme is present between the target gene coding unit and the RNA polymerase coding unit.

The target protein encoded by the replicon of the present disclosure may also be an immunogenic compound or an antigenic epitope or cytokine for therapeutic or protective purposes. The term "antigen" or "immunogen" refers to a substance capable of eliciting the production of antibodies, any substance capable of eliciting an immune response. Antigens are preferably products derived from naturally occurring antigens, such as from allergens, viruses, bacteria, fungi, parasites and other infectious agents and pathogens, and tumor antigens. The term "cytokine" refers to a class of small molecular proteins with a wide range of biological activities that are synthesized and secreted by immune cells (such as monocytes, macrophages, T cells, B cells, NK cells, etc.) and certain non-immune cells (endothelial cells, epidermal cells, fibroblasts, etc.) upon stimulation, including interleukins, interferons, tumor necrosis factor superfamily, colony-stimulating factors, chemokines, growth factors, etc.

When a biological entity (such as replicase, virus, subgenomic promoter (SGP)) is used herein, the meaning includes not only its wild-type form, but also variant forms that retain the function of the biological entity.

### Safety features of embodiments of the present disclosure

Preferably, the system of the present disclosure does not contain any alphavirus structural proteins, such as capsid protein C, membrane glycoproteins E1, E2 and E3, and 6K protein.

Preferably, after the host cell is inoculated with the cis-replicon RNA of the present disclosure, the host cell does not produce virus particles, e.g., progeny virus particles.

Preferably, the replicon cannot drive the replication of the replicase itself, but can only drive the replication of the target gene RNA.

### DNA of the present disclosure

The present disclosure provides DNA encoding the replicon of the present disclosure. In a preferred embodiment, the DNA provided by the present disclosure is a plasmid. As used herein, the term "plasmid" refers to a DNA molecule outside the chromosome (or nucleoid) in organisms such as bacteria, yeast and actinomycetes, which has the ability to replicate autonomously and expresses the genetic information it carries, and is a closed circular double-stranded DNA molecules.

### Methods for preparing RNA

Any RNA molecule according to the present disclosure can be obtained by in vitro transcription. In vitro transcribed RNA (IVT-RNA) can be obtained by in vitro transcription from nucleic acid molecules, especially DNA molecules. The 5' cap can be obtained by adding a cap-analogue to an in vitro transcription reaction, and the poly(A) tail is encoded by a poly(dT) sequence on the DNA template. Alternatively, capping and poly(A) tailing can also be achieved by enzymatic reactions after the transcripts are obtained. In vitro transcription methods are known to those skilled in the art, and various in vitro transcription kits are commercially available.

### Vaccine Adjuvant

In some embodiments, the immunostimulatory properties of mRNA can be improved by adding adjuvants to enhance the efficacy of mRNA vaccines. These approaches include using traditional adjuvants as well as novel approaches that exploit the inherent immunogenicity of mRNA or the immunomodulatory proteins it encodes. The compositions of the present disclosure may comprise one or more adjuvants.

Adjuvants can be added to vaccines to stimulate an immune system response. Exemplary adjuvants include but are not limited to biological adjuvants such as mycobacteria (mycobacterium tuberculosis, bacille Calmette-Guérin (BCG)), corynebacterium parvum, bordetella pertussis, gram-negative bacillus endotoxin, interleukin-1, interleukin-2, interleukin-12, interferon-γ, etc.; inorganic adjuvants such as aluminum hydroxide, alum, aluminum phosphate, etc.; synthetic adjuvants such as double-chain polyinosinic acid, cytidylic acid, double-chain polyadenosine acid, etc.; oil agents such as peanut oil emulsified adjuvant, mineral oil, vegetable oil, lanolin, etc.; and immune activation proteins such as CD70, CD40L and TLR4, etc.

### RNA drug delivery system

Cell membranes are mainly composed of a lipid bilayer of zwitterionic and negatively charged phospholipids, where the polar heads of the phospholipids point towards the aqueous environment and the hydrophobic tails form the hydrophobic core. Various ion pumps and ion channels help maintain a negative potential (-40 to -80 mV) across the cell membrane to control the balance of most essential metal ions such as K⁺, Na⁺, Ca²⁺ and Mg²⁺. RNA must cross the cell membrane to reach the cytoplasm, and the negative potential across the cell membrane creates a strong barrier for highly negatively charged RNA molecules. In addition to the cell membrane barrier, RNA also faces the problem of degradation of extracellular ribonucleic acid present in large quantities in the skin and blood. Naked RNA molecules are rapidly degraded in organisms, do not accumulate in target tissues after systemic administration, and cannot penetrate target cells even if they reach target tissues, so a delivery system is required.

In an embodiment, the RNA pharmaceutical composition of the present disclosure comprises at least one cation-containing complex. The pharmaceutical composition according to the present disclosure can be encapsulated in polyplex (PP), lipoplex (LP), lipid nanoparticle (LNP), or cationic nanoemulsion (CNE).

In a preferred embodiment of the present disclosure, polymer is, for example, polyplex (PP), which is formed by electrostatic interaction of polyethyleneimine (PEI) with negatively charged nucleic acids. PEI is a group of linear or branched polyethylenimine polymers that have a strong affinity for nucleic acids and have a proton sponge effect that promotes endosomal escape, protecting nucleic acids from enzymatic degradation (Thomas Démoulins et al., Nanomedicine, 2016, Vol. 12, pp. 711-722).

In a preferred embodiment of the present disclosure, negatively charged mRNA and positively charged cationic liposomes are assembled through electrostatic interaction to form a multilayer vesicular complex, namely lipoplex (LP), in which the encapsulated mRNA is not easily degraded by RNase and can be successfully delivered into cells. DOTAP (1,2-dioleoyloxy-3-(trimethylammonium)propane), DOPE (1,2-dioleoyl-sn-glycero-3-phosphoethanolamine) and DOTMA (N-(1-(2,3-dioleyloxy)propyl)-N,N,N-trimethylammonium) are proven effective delivery vehicles. Cationic lipids are commonly used in mRNA vaccinations because they not only encapsulate mRNA, but can also be used as immunogens and are considered as adjuvants for vaccines (Yasmin Granot et al., Seminars in Immunology, 2017, Vol. 34, No. pp. 68-77).

In a preferred embodiment of the present disclosure, the mRNA is present in a lipid nanoparticle (LNP), which typically comprises (1) an ionizable or cationic lipid or polymeric material with tertiary amines or quaternary amines, which can promote self-assembly into virus-sized (-100 nm) particles, is used to encapsulate polyanionic mRNA and allows endosomes to release mRNA into the cytoplasm; (2) an auxiliary phospholipid, which is generally a zwitterionic lipid, such as DSPC (1,2-distearoyl-sn-glycero-3-phosphocholine), DOPE (1,2-dioleoyl-sn-glycero-3-phosphoethanolamine) or POPE (1-palmitoyl-2-oleoylphosphatidylethanolamine). Auxiliary phospholipids serve as the backbone of liposomes, similar to lipids in cell membranes, and contribute to the formation and disruption of lipid bilayers to facilitate endosomal escape. Some phospholipids have polymorphic characteristics, which can change from lamellar to hexagonal phase when entering endosomes, and promote the release of mRNA from liposomes; (3) cholesterol, to stabilize the lipid bilayer of LNP and improve particle stability, which enhances the stability of particles and promotes membrane fusion; (4) lipid-linked polyethylene glycol (PEG), making nanoparticles have a hydration layer and improves colloidal stability, which can reduce non-specific interactions with serum proteins, circumvent absorption through the reticuloendothelial system and prolong the half-life of formulations (Norbert Pardi et al., 2018, Nat Rev Drug Discov., Vol. 17, pp. 261-279; Piotr S. Kowalski et al., Mol Ther, 2019, p. 27 vol., pp. 710-728).

In a preferred embodiment of the present disclosure, the mRNA is present in a cationic nanoemulsion (CNE) made of squalene, nonionic sorbitan ester surfactant, sorbitan trioleate (Span 85) or sorbitan monostearate (Span 60), cationic lipid DOTAP, etc., which can be used in the preparation of vaccines (Jesse H. Erasmus et al., Mol Ther., 2018, Vol. 26, pp. 2507-2522).

### Methods for producing protein

The present disclosure provides a method for producing a protein in a cell comprising steps of: obtaining an RNA construct for expressing an exogenous protein comprising a 5' cap for driving translation of the replicase, a 3'poly(A) for enhancing mRNA stability, and an open reading frame encoding the exogenous protein, and transfecting the RNA construct into the cell.

In the method of the present disclosure, any system according to the present disclosure, or a kit according to the present disclosure, or a pharmaceutical composition according to the present disclosure may be used. The RNA can be used in the form of a pharmaceutical composition, or as naked RNA, for example, for electroporation or lipofection, and the like.

The present disclosure provides a method for producing a protein in an organism, comprising the steps of: obtaining an RNA construct for expressing an exogenous protein comprising a 5' cap for driving translation of the replicase, a 3' poly(A) for enhancing mRNA stability, and an open reading frame encoding the exogenous protein, and administering to a subject in need thereof the RNA construct. The medicaments of the present disclosure can be used in prophylactic as well as therapeutic methods of treating a subject.

### Vaccination

The term "immunization" or "vaccination" generally refers to the process of treating a subject for therapeutic or prophylactic reasons. Treatment, especially prophylactic treatment, is preferably or is intended to include treatment aimed at inducing or enhancing an immune response in a subject against one or more antigens. According to the present disclosure, if it is desired to induce or enhance an immune response by using RNA as described herein, the immune response may be elicited or enhanced by RNA. In one embodiment, the present disclosure provides prophylactic treatment, which preferably is or includes vaccination of a subject. One embodiment of the present disclosure is particularly suitable for vaccination, wherein the replicon encodes as target protein a pharmaceutically active peptide or protein, which is an immunologically active compound or antigen. The medicaments of the present disclosure can be administered to a subject, e.g., for treatment of a subject, including vaccination of a subject. The term "subject" relates to vertebrates, especially mammals including humans.

### Administration method

A medicament of the present disclosure may be administered to a subject by any suitable route.

For example, the medicament could be administered systemically, and administration can be performed in various ways, such as intravenously, subcutaneously, intradermally, intramuscularly or by inhalation. Alternatives for administration to muscle tissue or skin include, but are not limited to, intradermal, intranasal, intraocular, intraperitoneal, intravenous, interstitial, buccal, transdermal or sublingual administration, and the like.

### Examples

### I. Materials and Methods

### (1) Construction of DNA vectors carrying a trans-replicon, a traditional cis-replicon and a cis-replicon of the present disclosure

The sequences of some elements used in the example are as follows:
5' cap structure: 7-methylguanosine cap structure (m7Gppp, Cap 0)
5' UTR (derived from human alpha globin): actcttctggtccccacagactcagagagaacccacc (SEQ ID NO: 1)
CSE1 (derived from Semliki Forest Virus (SFV) of the alphavirus genus):
   atggcggatgtgtgacatacacgacgccaaaagattttgttccag (SEQ ID NO: 2)
CSE2 (derived from Semliki Forest Virus (SFV) of the alphavirus genus):
   gcaggtcacaccaaatgaccatgcaaatgccagagcattttcgcacctggc (SEQ ID NO: 3)
SGP (CSE3) (derived from Semliki Forest Virus (SFV) of the alphavirus genus):
   acctctacggcggtcctag (SEQ ID NO: 4)
CSE4 (derived from Semliki Forest Virus (SFV) of the alphavirus genus):
   attggtttttaatatttcc (SEQ ID NO: 5)
3' UTR (derived from Semliki Forest Virus (SFV) of the alphavirus genus): 
gttagggtaggcaatggcattgatatagcaagaaaattgaaaacagaaaaagttagggtaagcaatggcatataaccataact
gtataacttgtaacaaagcgcaacaagacctgcgcaattggccccgtggtccgcctcacggaaactcggggcaactcatattgacacattaa
ttggcaataattggaagcttacataagcttaattcgacgaataattggatttttattttattttgcaattggtttttaatatttcc (SEQ ID NO: 6)
Replicase (derived from Semliki Forest Virus (SFV) of the alphavirus genus), its amino acid sequence is:

MAAKVHVDIEADSPFIKSLQKAFPSFEVESLQVTPNDHANARAFSHLATKLIEQ
ETDKDTLILDIGSAPSRRMMSTHKYHCVCPMRSAEDPERLVCYAKKLAAASGKVLDREI
AGKITDLQTVMATPDAESPTFCLHTDVTCRTAAEVAVYQDVYAVHAPTSLYHQAMKGV
RTAYWIGFDTTPFMFDALAGAYPTYATNWADEQVLQARNIGLCAASLTEGRLGKLSILRK
KQLKPCDTVMFSVGSTLYTESRKLLRSWHLPSVFHLKGKQSFTCRCDTIVSCEGYVVKK
ITMCPGLYGKTVGYAVTYHAEGFLVCKTTDTVKGERVSFPVCTYVPSTICDQMTGILATD
VTPEDAQKLLVGLNQRIVVNGRTQRNTNTMKNYLLPIVAVAFSKWAREYKADLDDEKPL
GVRERSLTCCCLWAFKTRKMHTMYKKPDTQTIVKVPSEFNSFVIPSLWSTGLAIPVRSRI
KMLLAKKTKRELIPVLDASSARDAEQEEKERLEAELTREALPPLVPIAPAETGVVDVDVE
ELEYHAGAGVVETPRSALKVTAQPNDVLLGNYVVLSPQTVLKSSKLAPVHPLAEQVKII
THNGRAGRYQVDGYDGRVLLPCGSAIPVPEFQALSESATMVYNEREFVNRKLYHIAVHG
PSLNTDEENYEKVRAERTDAEYVFDVDKKCCVKREEASGLVLVGELTNPPFHEFAYEGL
KIRPSAPYKTTVVGVFGVPGSGKSAIIKSLVTKHDLVTSGKKENCQEIVNDVKKHRGLDI
QAKTVDSILLNGCRRAVDILYVDEAFACHSGTLLALIALVKPRSKVVLCGDPKQCGFFN

MMQLKVNFNHNICTEVCHKSISRRCTRPVTAIVSTLHYGGKMRTTNPCNKPIIIDTTGQT
KPKPGDIVLTCFRGWVKQLQLDYRGHEVMTAAASQGLTRKGVYAVRQKVNENPLYAPA
SEHVNVLLTRTEDRLVWKTLAGDPWIKVLSNIPQGNFTATLEEWQEEHDKIMKVIEGPAA
PVDAFQNKANVCWAKSLVPVLDTAGIRLTAEEWSTIITAFKEDRAYSPVVALNEICTKYY
GVDLDSGLFSAPKVSLYYENNHWDNRPGGRMYGFNAATAARLEARHTFLKGQWHTGK
QAVIAERKIQPLSVLDNVIPINRRLPHALVAEYKTVKGSRVEWLVNKVRGYHVLLVSEYN
LALPRRRVTWLSPLNVTGADRCYDLSLGLPADAGRFDLVFVNIHTEFRIHHYQQCVDHA
MKLQMLGGDALRLLKPGGSLLMRAYGYADKISEAVVSSLSRKFSSARVLRPDCVTSNTE
VFLLFSNFDNGKRPSTLHQMNTKLSAVYAGEAMHTAGCAPSYRVKRADIATCTEAAVVN
AANARGTVGDGVCRAVAKKWPSAFKGEATPVGTIKTVMCGSYPVIHAVAPNFSATTEAE
GDRELAAVYRAVAAEVNRLSLSSVAIPLLSTGVFSGGRDRLQQSLNHLFTAMDATDADV
TIYCRDKSWEKKIQEAIDMRTAVELLNDDVELTTDLVRVHPDSSLVGRKGYSTTDGSLYS
YFEGTKFNQAAIDMAEILTLWPRLQEANEQICLYALGETMDNIRSKCPVNDSDSSTPPRT
VPCLCRYAMTAERIARLRSHQVKSMVVCSSFPLPKYHVDGVQKVKCEKVLLFDPTVPSV
VSPRKYAASTTDHSDRSLRGFDLDWTTDSSSTASDTMSLPSLQSCDIDSIYEPMAPIVVTA
DVHPEPAGIADLAADVHPEPADHVDLENPIPPPRPKRAAYLASRAAERPVPAPRKPTPAPR
TAFRNKLPLTFGDFDEHEVDALASGITFGDFDDVLRLGRAGAYIFSSDTGSGHLQQKSVR
QHNLQCAQLDAVEEEKMYPPKLDTEREKLLLLKMQMHPSEANKSRYQSRKVENMKAT
VVDRLTSGARLYTGADVGRIPTYAVRYPRPVYSPTVIERFSSPDVAIAACNEYLSRNYPTV
ASYQITDEYDAYLDMVDGSDSCLDRATFCPAKLRCYPKHHAYHQPTVRSAVPSPFQNTL
QNVLAAATKRNCNVTQMRELPTMDSAVFNVECFKRYACSGEYWEEYAKQPIRITTENIT
TYVTKLKGPKAAALFAKTHNLVPLQEVPMDRFTVDMKRDVKVTPGTKHTEERPKVQVI
QAAEPLATAYLCGIHRELVRRLNAVLRPNVHTLFDMSAEDFDAIIASHFHPGDPVLETDIA
SFDKSQDDSLALTGLMILEDLGVDQYLLDLIEAAFGEISSCHLPTGTRFKFGAMMKSGMF
LTLFINTVLNITIASRVLEQRLTDSACAAFIGDDNIVHGVISDKLMAERCASWVNMEVKII
DAVMGEKPPYFCGGFIVFDSVTQTACRVSDPLKRLFKLGKPLTAEDKQDEDRRRALSDE
VSKWFRTGLGAELEVALTSRYEVEGCKSILIAMATLARDIKAFKKLRGPVIHLYGGPRLV
R (SEQ ID NO: 7)

(1) The DNA sequences of each fragment required for the construction of the replicon in the present disclosure were synthesized by Sangon Bioengineering (Shanghai) Co., Ltd., and inserted into the mammalian expression vector pVAX1 (purchased from Invitrogen) by homologous recombination to construct pVAX1-cis-SFV-neo cis-replicon plasmid (the structure is shown in FIG. 2A, where the position of the target gene is a multiple cloning site). The traditional cis-replicon carrying the same polymerase (the structure is shown in FIG. 1B, where the position of the target gene is a multiple cloning site) and trans-replicon system (the structure is shown in FIG. 1C, where the position of the target gene is a multiple cloning site point) were synthesized and constructed according to the disclosure of patent publication CN 109328233 A, and inserted into the mammalian expression vector pVAX1 by homologous recombination, named pVAX1-trans-SFV and pVAX1-cis-SFV, respectively.
   The pVAX1-cis-SFV-neo obtained after construction contains the following sequence:
   5' UTR: actcttctggtccccacagactcagagagaacccacc (SEQ ID NO: 8)
   NSP1: 
atggccgccaaagtgcatgttgatattgaggctgacagcccattcatcaagtctttgcagaaggcatttccgtcgttcgaggtggagtcattgc
aggtcacaccaaatgaccatgcaaatgccagagcattttcgcacctggctaccaaattgatcgagcaggagactgacaaagacacactcat
cttggatatcggcagtgcgccttccaggagaatgatgtctacgcacaaataccactgcgtatgccctatgcgcagcgcagaagaccccgaa
aggctcgtatgctacgcaaagaaactggcagcggcctccgggaaggtgctggatagagagatcgcaggaaaaatcaccgacctgcaga
ccgtcatggctacgccagacgctgaatctcctaccttttgcctgcatacagacgtcacgtgtcgtacggcagccgaagtggccgtataccag
gacgtgtatgctgtacatgcaccaacatcgctgtaccatcaggcgatgaaaggtgtcagaacggcgtattggattgggtttgacaccacccc
gtttatgtttgacgcgctagcaggcgcgtatccaacctacgccacaaactgggccgacgagcaggtgttacaggccaggaacataggact
gtgtgcagcatccttgactgagggaagactcggcaaactgtccattctccgcaagaagcaattgaaaccttgcgacacagtcatgttctcggt
aggatctacattgtacactgagagcagaaagctactgaggagctggcacttaccctccgtattccacctgaaaggtaaacaatcctttacctgt
aggtgcgataccatcgtatcatgtgaagggtacgtagttaagaaaatcactatgtgccccggcctgtacggtaaaacggtagggtacgccgt
gacgtatcacgcggagggattcctagtgtgcaagaccacagacactgtcaaaggagaaagagtctcattccctgtatgcacctacgtcccct
caaccatctgtgatcaaatgactggcatactagcgaccgacgtcacaccggaggacgcacagaagttgttagtgggattgaatcagaggat
agttgtgaacggaagaacacagcgaaacactaacacgatgaagaactatctgcttccgattgtggccgtcgcatttagcaagtgggcgagg
gaatacaaggcagaccttgatgatgaaaaacctctgggtgtccgagagaggtcacttacttgctgctgcttgtgggcatttaaaacgaggaa
gatgcacaccatgtacaagaaaccagacacccagacaatagtgaaggtgccttcagagtttaactcgttcgtcatcccgagcctatggtcta
caggcctcgcaatcccagtcagatcacgcattaagatgcttttggccaagaagaccaagcgagagttaatacctgttctcgacgcgtcgtca
gccagggatgctgaacaagaggagaaggagaggttggaggccgagctgactagagaagccttaccacccctcgtccccatcgcgccgg
cggagacgggagtcgtcgacgtcgacgttgaagaactagagtatcacgcaggtgca (SEQ ID NO: 9)
   NSP2: 
ggggtcgtggaaacacctcgcagcgcgttgaaagtcaccgcacagccgaacgacgtactactaggaaattacgtagttctgtccccgcag
accgtgctcaagagctccaagttggcccccgtgcaccctctagcagagcaggtgaaaataataacacataacgggagggccggccgttac
caggtcgacggatatgacggcagggtcctactaccatgtggatcggccattccggtccctgagtttcaagctttgagcgagagcgccactat
ggtgtacaacgaaagggagttcgtcaacaggaaactataccatattgccgttcacggaccgtcgctgaacaccgacgaggagaactacga
gaaagtcagagctgaaagaactgacgccgagtacgtgttcgacgtagataaaaaatgctgcgtcaagagagaggaagcgtcgggtttggt
gttggtgggagagctaaccaacccccccgttccatgaattcgcctacgaagggctgaagatcaggccgtcggcaccatataagactacagta
gtaggagtctttggggttccgggatcaggcaagtctgctattattaagagcctcgtgaccaaacacgatctggtcaccagcggcaagaagg
agaactgccaggaaatagtcaacgacgtgaagaagcaccgcggactggacatccaggcaaaaacagtggactccatcctgctaaacgg
 
gtgtcgtcgtgccgtggacatcctatatgtggacgaggctttcgcttgccattccggtactctgctagccctaattgctcttgttaaacctcgga
gcaaagtggtgttatgcggagaccccaagcaatgcggattcttcaatatgatgcagcttaaggtgaacttcaaccacaacatctgcactgaa
gtatgtcataaaagtatatccagacgttgcacgcgtccagtcacggccatcgtgtctacgttgcactacggaggcaagatgcgcacgaccaa
cccgtgcaacaaacccataatcatagacaccacaggacagaccaagcccaagccaggagacatcgtgttaacatgcttccgaggctgggt
aaagcagctgcagttggactaccgtggacacgaagtcatgacagcagcagcatctcagggcctcacccgcaaaggggtatacgccgtaa
ggcagaaggtgaatgaaaatcccttgtatgcccctgcgtcggagcacgtgaatgtactgctgacgcgcactgaggataggctggtgtggaa
aacgctggccggcgatccctggattaaggtcctatcaaacattccacagggtaactttacggccacattggaagaatggcaagaagaacac
gacaaaataatgaaggtgattgaaggaccggctgcgcctgtggacgcgttccagaacaaagcgaacgtgtgttgggcgaaaagcctggt
gcctgtcctggacactgccggaatcagattgacagcagaggagtggagcaccataattacagcatttaaggaggacagagcttactctcca
gtggtggccttgaatgaaatttgcaccaagtactatggagttgacctggacagtggcctgttttctgccccgaaggtgtccctgtattacgaga
acaaccactgggataacagacctggtggaaggatgtatggattcaatgccgcaacagctgccaggctggaagctagacataccttcctgaa
ggggcagtggcatacgggcaagcaggcagttatcgcagaaagaaaaatccaaccgctttctgtgctggacaatgtaattcctatcaaccgc
aggctgccgcacgccctggtggctgagtacaagacggttaaaggcagtagggttgagtggctggtcaataaagtaagagggtaccacgtc
ctgctggtgagtgagtacaacctggctttgcctcgacgcagggtcacttggttgtcaccgctgaatgtcacaggcgccgataggtgctacga
cctaagtttaggactgccggctgacgccggcaggttcgacttggtctttgtgaacattcacacggaattcagaatccaccactaccagcagtg
tgtcgaccacgccatgaagctgcagatgcttgggggagatgcgctacgactgctaaaacccggcggcagcctcttgatgagagcttacgg
atacgccgataaaatcagcgaagccgttgtttcctccttaagcagaaagttctcgtctgcaagagtgttgcgcccggattgtgtcaccagcaat
acagaagtgttcttgctgttctccaactttgacaacggaaagagaccctctacgctacaccagatgaataccaagctgagtgccgtgtatgcc
ggagaagccatgcacacggccgggtgt (SEQ ID NO: 10)
   NSP3: 
gcaccatcctacagagttaagagagcagacatagccacgtgcacagaagcggctgtggttaacgcagctaacgcccgtggaactgtagg
ggatggcgtatgcagggccgtggcgaagaaatggccgtcagcctttaagggagaagcaacaccagtgggcacaattaaaacagtcatgt
gcggctcgtaccccgtcatccacgctgtagcgcctaatttctctgccacgactgaagcggaaggggaccgcgaattggccgctgtctaccg
ggcagtggccgccgaagtaaacagactgtcactgagcagcgtagccatcccgctgctgtccacaggagtgttcagcggcggaagagata
ggctgcagcaatccctcaaccatctattcacagcaatggacgccacggacgctgacgtgaccatctactgcagagacaaaagttgggaga
agaaaatccaggaagccatagacatgaggacggctgtggagttgctcaatgatgacgtggagctgaccacagacttggtgagagtgcacc
cggacagcagcctggtgggtcgtaagggctacagtaccactgacgggtcgctgtactcgtactttgaaggtacgaaattcaaccaggctgc
tattgatatggcagagatactgacgttgtggcccagactgcaagaggcaaacgaacagatatgcctatacgcgctgggcgaaacaatgga
caacatcagatccaaatgtccggtgaacgattccgattcatcaacacctcccaggacagtgccctgcctgtgccgctacgcaatgacagca
gaacggatcgcccgccttaggtcacaccaagttaaaagcatggtggtttgctcatcttttcccctcccgaaataccatgtagatggggtgcag
aaggtaaagtgcgagaaggttctcctgttcgacccgacggtaccttcagtggttagtccgcggaagtatgccgcatctacgacggaccactc
agatcggtcgttacgagggtttgacttggactggaccaccgactcgtcttccactgccagcgataccatgtcgctacccagtttgcagtcgtgt
gacatcgactcgatctacgagccaatggctcccatagtagtgacggctgacgtacaccctgaacccgcaggcatcgcggacctggcggc
agatgtgcatcctgaacccgcagaccatgtggacctcgagaacccgattcctccaccgcgcccgaagagagctgcataccttgcctcccg
cgcggcggagcgaccggtgccggcgccgagaaagccgacgcctgccccaaggactgcgtttaggaacaagctgcctttgacgttcggc
 
gactttgacgagcacgaggtcgatgcgttggcctccgggattactttcggagacttcgacgacgtcctgcgactaggccgcgcgggtgca
(SEQ ID NO: 11)
   NSP4: 
tatattttctcctcggacactggcagcggacatttacaacaaaaatccgttaggcagcacaatctccagtgcgcacaactggatgcggtcgag
gaggagaaaatgtacccgccaaaattggatactgagagggagaagctgttgctgctgaaaatgcagatgcacccatcggaggctaataag
agtcgataccagtctcgcaaagtggagaacatgaaagccacggtggtggacaggctcacatcgggggccagattgtacacgggagcgg
acgtaggccgcataccaacatacgcggttcggtaccccgccccgtgtactcccctaccgtgatcgaaagattctcaagccccgatgtagc
aatcgcagcgtgcaacgaatacctatccagaaattacccaacagtggcgtcgtaccagataacagatgaatacgacgcatacttggacatg
gttgacgggtcggatagttgcttggacagagcgacattctgcccggcgaagctccggtgctacccgaaacatcatgcgtaccaccagccg
actgtacgcagtgccgtcccgtcaccctttcagaacacactacagaacgtgctagcggccgccaccaagagaaactgcaacgtcacgcaa
atgcgagaactacccaccatggactcggcagtgttcaacgtggagtgcttcaagcgctatgcctgctccggagaatattgggaagaatatgc
taaacaacctatccggataaccactgagaacatcactacctatgtgaccaaattgaaaggcccgaaagctgctgccttgttcgctaagaccc
acaacttggttccgctgcaggaggttcccatggacagattcacggtcgacatgaaacgagatgtcaaagtcactccagggacgaaacaca
cagaggaaagacccaaagtccaggtaattcaagcagcggagccattggcgaccgcttacctgtgcggcatccacagggaattagtaagg
agactaaatgctgtgttacgccctaacgtgcacacattgtttgatatgtcggccgaagactttgacgcgatcatcgcctctcacttccacccag
gagacccggttctagagacggacattgcatcattcgacaaaagccaggacgactccttggctcttacaggtttaatgatcctcgaagatctag
gggtggatcagtacctgctggacttgatcgaggcagcctttggggaaatatccagctgtcacctaccaactggcacgcgcttcaagttcgga
gctatgatgaaatcgggcatgtttctgactttgtttattaacactgttttgaacatcaccatagcaagcagggtactggagcagagactcactga
ctccgcctgtgcggccttcatcggcgacgacaacatcgttcacggagtgatctccgacaagctgatggcggagaggtgcgcgtcgtgggt
caacatggaggtgaagatcattgacgctgtcatgggcgaaaaacccccatatttttgtggggggattcatagtttttgacagcgtcacacagac
cgcctgccgtgtttcagacccacttaagcgcctgttcaagttgggtaagccgctaacagctgaagacaagcaggacgaagacaggcgacg
agcactgagtgacgaggttagcaagtggttccggacaggcttgggggccgaactggaggtggcactaacatctaggtatgaggtagagg
gctgcaaaagtatcctcatagccatggccaccttggcgagggacattaaggcgtttaagaaattgagaggacctgttatacacctctatggag
gaccccgtctagtaaggtga (SEQ ID NO: 12)
   CSE1: atggcggatgtgtgacatacacgacgccaaaagattttgttccag (SEQ ID NO: 13)
   CSE2: gcaggtcacaccaaatgaccatgcaaatgccagagcattttcgcacctggc (SEQ ID NO: 14)
   CSE3: acctctacggcggtcctag (SEQ ID NO: 15)
   Multiple cloning site (MCS): atcgatatgcatttataaggatcccctaggcgatcg (SEQ ID NO: 16)
   3' UTR: 
gttagggtaggcaatggcattgatatagcaagaaaattgaaaacagaaaaagttagggtaagcaatggcatataaccataactgtataacttg
taacaaagcgcaacaagacctgcgcaattggccccgtggtccgcctcacggaaactcggggcaactcatattgacacattaattggcaata
attggaagcttacataagcttaattcgacgaataattggatttttattttattttgca (SEQ ID NO: 17)
   CSE4: attggtttttaatatttcc (SEQ ID NO: 18)
      PolyA: aaaaaaaaaaaaaaaaaaaaaaaaaaaaaaaaaaaaaaaaaaaaaaaaaaaaaaaaaaaaaaaaaaaaa (SEQ ID NO: 19)
(2) The enhanced green fluorescent protein (EGFP) coding region or the luciferase reporter gene (Luc) were homologously recombined into the multiple cloning site region at the 3' end of the subgenomic promoter of the modified expression vector pVAX1, the traditional cis-replicon vector pVAX1-cis-SFV, the trans-replicon vector pVAX1-trans-SFV, and the cis-replicon vector pVAX1-cis-SFV-neo of the present disclosure, to construct pVAX1-EGFP/Luc, pVAX1-cis-SFV-EGFP/Luc, pVAX1-trans-SFV-EGFP/Luc, and pVAX1-cis-SFV-neo-EGFP/Luc, respectively.
(3) The receptor binding domain (RBD) in the spike protein of SARS-CoV-2 was homologously recombined into the modified expression vector pVAX1 and the cis-replicon pVAX1-cis-SFV-neo by inserting into the multiple cloning site region at the 3' end of the subgenomic promoter to construct pVAX1-RBD and pVAX1-cis-SFV-neo-RBD.
(4) The vectors obtained from the construction described in (1)-(3) were subjected to in vitro transcription using the HiScribe^{™} T7 ARCA mRNA Kit (with Tailing) (New England Biolabs, MA, USA) according to the instructions, and then RNA was collected to detect the RNA concentration by Nanodrop 2000 ultra-micro spectrophotometer (Thermo Fisher Scientific, MA, USA). The prepared RNA was stored in a -80 degree refrigerator for later use.
(5) RNA transfection
   All cell culture media, fetal bovine serum (FBS), antibiotics and other supplements in this example were provided by Thermo Fisher Scientific, USA. The RNA obtained from *in vitro* transcription was subjected to RNA liposome transfection using the transfection reagent Lipofectamine Messenger MAX (Thermo Fisher Scientific, MA, USA) according to the manufacturer's instructions.
(6) Detection of green fluorescent protein signal
   In order to evaluate the level of exogenous protein (green fluorescent protein) expressed by different RNA replicons, the RNA obtained from *in vitro* transcription of pVAX1-EGFP (without replicons, as a control), pVAX1-cis-SFV-EGFP, pVAX1-trans-SFV-EGFP, and pVAX1-cis-SFV-neo-EGFP according to the method described in (5) were transfected into hamster kidney fibroblasts (BHK-21) (purchased from Cell Bank of Type Culture Collection Committee, Chinese Academy of Sciences) in 96-well plate in equal amounts. After 48 hours, the green fluorescence intensity was observed under the microscope and the images were recorded.
(7) Luciferase activity assay
   In order to evaluate the level of exogenous protein (luciferase) expressed by different RNA replicons, the RNA obtained by in vitro transcription of pVAX1-Luc (without replicons, as a control), pVAX1-cis-SFV-Luc, pVAX1-trans-SFV-Luc, and pVAX1-cis-SFV-neo-Luc according to the method described in (5) were transfected into BHK-21 cells in 96-well plate in equal amounts. The firefly luciferase was detected using Bright-Glo (Promega, Madison, USA) detection reagent, and bioluminescence was measured using an EnSpire multi-function microplate reader (PerkinElmer, MA, USA). Data were expressed as relative luciferase units [RLU], background signal (readout from untransfected cells) subtracted.
(8) Detection of protein expression
   The RNA obtained by in vitro transcription of pVAX1-RBD and pVAX1-cis-SFV-neo-RBD were transfected into BHK-21 cells in 24-well plate in equal amounts, and the cells were collected 48 hours later. The expression of RBD of SARS-CoV-2 was detected and analyzed by Western Blot, and the antibody used was the RBD antibody against SARS-CoV-2 produced by Beijing SinoBiological Co., Ltd.
(9) Pseudovirus neutralization assay
   A pseudotyped lentiviral luciferase reporter system was used to evaluate the neutralization ability of sera from inoculated animals against SARS-CoV-2.

Specifically, the SARS-CoV-2 pseudotyped virus was produced by co-transfecting 293T cells with the lentiviral packaging plasmid pCMV delta R8.2, the expression plasmid pCDH-CMV-luc encoding the luciferase reporter gene, and a plasmid pcDNA3.1-Spike expressing the Spike protein of SARS-CoV-2, and the treatment ratio for transfection was 1:1.5:1. The virus-containing medium was collected by centrifugation, filtered through a 0.45 µm membrane, and then stored at -80 °C for future use.

For the neutralization assay, serial dilutions of the heat-inactivated sera to be tested were first incubated with SARS-CoV-2 pseudotyped virus for 1 hour, and then the serum-virus mixture was transferred to 293T-hACE2 cells pre-seeded on cell culture plates. Cells were lysed after 48 h, and luciferase activity was determined with Bright-Glo (Promega, Madison, USA) detection reagent. IC50 neutralization titers were then calculated using GraphPad Prism (version 8.4).

### (10) Immunization of mice

BALB/c mice aged 6-8 weeks were purchased from the Experimental Animal Center of Henan Province and raised in an SPF level animal laboratory. All experiments were approved by the Life Science Ethics Review Committee of Zhengzhou University.

The mice were injected intramuscularly with the liposome-encapsulated RNA obtained by in vitro transcription of pVAX1-RBD (control) and pVAX1-cis-SFV-neo-RBD (experimental group), and boosted with equal doses of RNA on days 14 and 28 after the initial immunization. Serum samples were collected 35 days after the initial immunization, heat-inactivated at 56°C for 30 min, and stored at -80°C. The control group of immunized mice was injected with PBS, or liposome-encapsulated RNA obtained by in vitro transcription of pVAX1 plasmid. Five mice were used in both the experimental group and the control group, and the experimental results were expressed as mean ± SEM. The comparison between groups was performed by the Mann-Whitney U test, and statistical analysis was performed by GraphPad Prism (version 8.4) software.

### II. Results and Analysis

1. The RNA obtained by in vitro transcription of expression vector pVAX1-EGFP, the traditional cis-replicon vector pVAX1-cis-SFV-EGFP, the trans-replicon vector pVAX1-trans-SFV-EGFP (containing the corresponding replicase), and the cis-replicon vector pVAX1-cis-SFV-neo-EGFP of the present disclosure were transfected into BHK-21 cells in 96-well plate in equal amounts using Lipofectamine MessengerMAX^{™}. After 48 hours, the ability of various replicons to express green fluorescent protein (EGFP) was compared.
   The results are shown in FIG. 4 that the effects of the traditional cis-replicon (FIG. 4B), the trans-replicon (FIG. 4C) and the cis-replicon (FIG. 4D) of the present disclosure in expressing green fluorescent protein are far superior to those of RNA transcribed from traditional expression vectors (FIG. 4A).
2. The RNA obtained by transcription of the traditional expression vector pVAX1-Luc, the traditional cis-replicon pVAX1-cis-SFV-Luc, the trans-replicon pVAX1-trans-SFV-Luc (containing the corresponding replicase), and the cis-replicon pVAX1-cis-SFV-neo-Luc of the present disclosure were transfected into BHK-21 cells in 96-well plate in equal amounts using Lipofectamine MessengerMAX^{™}. After 48 hours, the luciferase activity was detected with the Bright-Glo luciferase assay system, and the ability of various replicons to express luciferase (Luc) was compared. The experimental results were expressed as mean ± SEM, and the comparison between groups was performed by the Mann-Whitney U test.
   The results are shown in FIG. 5 that the ability of the RNA transcribed from the traditional cis-replicon, trans-replicon (containing the corresponding replicase), and the cis-replicon vector of the present disclosure to express luciferase far exceeds that of the RNA transcribed from traditional expression vectors (P<0.01). The luciferase-expressing ability of the RNA transcribed from the cis-replicon vector of the present disclosure is equivalent to that of the trans-replicon (containing the corresponding replicase) vector, both significantly better than that of the traditional cis-replicon (P<0.01).
3. The RNA obtained by in vitro transcription of pVAX1-RBD/pVAX1-cis-SFV-neo-RBD was transfected using the transfection reagent Lipofectamine Messenger MAX into BHK-21 cells for 48 hours to detect the expression level. The results of Western Blot are shown in FIG. 6 that compared with the RNA transcribed from the traditional expression vector, the cis-replicon of the present disclosure expressed a higher amount of exogenous protein RBD.
4. The mice were immunized with 100 ng of RNA obtained by in vitro transcription of pVAX1-SFV-RBD and pVAX1-cis-SFV-neo-RBD after encapsulation in liposomes, and boosted with equal doses of RNA on days 14 and 28 after the initial immunization. Serum samples were collected 35 days after the initial immunization, and the anti-RBD IgG titers in the serum were measured by ELISA (coating antigen was recombinant RBD protein). In the control group, mice were immunized with PBS or mRNA in vitro transcribed from pVAX1. The experimental results were compared with the mean ± SEM of three independent experiments, and the comparison between groups was performed by the Mann-Whitney U test. In the ELISA experiment, the titer of the antiserum with no responses was designated as 1 for statistical analysis.
   The results are shown in FIG. 7 that after the mice were immunized with the RNA transcribed from the cis-replicon vector pVAX1-cis-SFV-neo-RBD of the present disclosure and the traditional expression vector pVAX1-RBD, they all produced IgG antibody targeting the receptor-binding domain (RBD) of SARS-CoV-2, and the antibody titer produced by the RNA replicon group transcribed in vitro from the cis-replicon vector of the present disclosure is significantly higher than that of the RNA group transcribed from the traditional expression vector (P<0.05).
5. The mice were immunized with 100 ng of RNA obtained by in vitro transcription of pVAX1-SFV-RBD and pVAX1-RBD after encapsulation in liposomes, and boosted with equal doses of RNA on days 14 and 28 after the initial immunization. Serum samples were collected 35 days after the initial immunization, and serum neutralization titers were determined by pseudovirus neutralization assay. In the control group, mice were immunized with PBS or RNA transcribed in vitro from pVAX1. The experimental results were compared with the mean ± SEM of three independent experiments, and the comparison between groups was performed by the Mann-Whitney U test. In the neutralization experiment, if the undiluted serum still did not show any neutralizing effect, its titer was assigned as 1 for statistical analysis.

The results are shown in FIG. 8 that in the *in vitro* pseudovirus neutralization assay, after the mice were immunized with the RNA transcribed from the cis-replicon vector pVAX1-cis-SFV-neo-RBD of the present disclosure or the expression vector pVAX1-RBD, the neutralizing antibodies were produced in the serum, providing protection to the cells. Meanwhile, the antibody titer of the cis-RNA replicon group of the present disclosure is significantly higher than that of the RNA group transcribed from the traditional expression vector pVAX1-RBD (P<0.05).

## Claims

1. An expression construct of cis-replicon, comprising an RNA polymerase coding unit and a target protein coding unit, wherein
the RNA polymerase coding unit comprises a nucleic acid fragment encoding an RNA polymerase, and the target protein coding unit comprises an insertion site (such as a multiple cloning site) for a target protein coding fragment and/or a nucleic acid fragment encoding the target protein; and
the construct is capable of expressing the RNA polymerase and replicating itself dependent on the RNA polymerase in a target cell to provide a template for expressing the target protein; preferably, the replication of the target protein coding unit is more efficiently than the replication of the RNA polymerase coding unit; and more preferably, only the target protein coding unit is replicated.

2. The expression construct according to claim 1, which is DNA or RNA, preferably RNA, including single-stranded RNA or double-stranded RNA, such as single-stranded RNA, particularly positive strand (+) single-stranded RNA.

3. The expression construct according to claim 1 or 2, which is linear or circular;
particularly, the construct is a linear RNA, wherein a 5' cap structure or an IRES sequence is provided in the 5' end of the RNA to drive translation of downstream coding sequence; or
the construct is a circular RNA, wherein an IRES sequence is provided upstream of the RNA polymerase coding unit to drive translation of the RNA polymerase coding unit.

4. The expression construct according to any one of claims 1 to 3, comprising replication initiating elements located both upstream and downstream of the target protein coding unit, wherein the replication initiating elements dependent on the RNA polymerase for the replication of the target protein coding unit; wherein the RNA polymerase coding unit is preferably not located between the upstream replication initiating element and downstream replication initiating element.

5. The expression construct according to claim 4, wherein the upstream replication initiating element comprises a CSE1 and/or CSE3 sequence, preferably CSE1 and CSE3 sequences, and more preferably CSE1, CSE2 and CSE3 sequences; and/or the downstream replication initiating element comprises a CSE4 sequence.

6. The expression construct according to any one of claims 1 to 5, wherein the target protein coding unit is located downstream or upstream of the RNA polymerase coding unit.

7. The expression construct according to any one of claims 1 to 6, wherein the target protein coding unit is located upstream of the RNA polymerase coding unit, and the target protein and the RNA polymerase are expressed as a fusion protein with a linker between the target protein and the RNA polymerase, wherein the linker comprises a fragment encoding a protease recognition site, and the RNA polymerase is released after the fusion protein is recognized and cleaved by a protease; and preferably, the protease recognition site is a 2A peptide sequence.

8. The expression construct according to any one of claims 1 to 6, wherein the target protein coding unit is located upstream of the RNA polymerase coding unit, and an IRES sequence is provided between the target protein coding unit and the RNA polymerase coding unit to drive translation of the RNA polymerase coding unit.

9. The expression construct according to any one of claims 1 to 6, wherein the target protein coding unit is located upstream of the RNA polymerase coding unit, and a self-cleaving HDV-like ribozyme is located between the target protein and the RNA polymerase.

10. The expression construct according to any one of claims 1 to 6, wherein the target protein coding unit is located downstream of the RNA polymerase coding unit, wherein the construct, in the 5'-3' direction, comprises a 5' cap structure or an IRES sequence to drive translation of downstream coding sequence, the RNA polymerase coding unit, the upstream replication initiating element, the target protein coding unit and the downstream replication initiating element.

11. The expression construct according to claim 10, wherein the construct, in the 5'-3' direction, comprises
(1) a 5' UTR sequence comprising a 5' cap structure;
(2) one or more nucleotide sequences encoding the RNA polymerase;
(3) a 5' end recognition sequence and a subgenomic promoter (SGP) that guide the replication of the RNA replicon;
(4) a multiple cloning site and/or a target gene or heterologous nucleotide sequence controlled by the subgenomic promoter;
(5) a 3' end recognition sequence that guides the replication of the RNA replicon; and
(6) a 3' UTR and 3' polyA that ensure the stability of RNA and the effective replication of the target gene.

12. The expression construct according to any one of claims 1 to 11, which is DNA, wherein a subgenomic promoter (SGP) is located upstream of the RNA polymerase coding unit.

13. The expression construct according to any one of claims 1 to 12, wherein the RNA polymerase is a viral replicase, particularly a replicase of an alphavirus,
preferably, the alphavirus is selected from the group consisting of Semliki Forest virus, Barmah Forest virus, Chikungunya virus, O'nyong'nyong virus, Ross River virus, Bebaru virus, Getah virus, Sagiyama virus, Mayaro virus, Una virus, Venezuelan equine encephalitis complex or Venezuelan equine encephalitis virus, Cabassou virus, Everglades virus, Mucambo virus, Tonate virus, Pixuna virus, Mosso das pedras virus, Rio Negro virus, Western equine encephalitis complex or Western equine encephalitis virus, Sindbis virus, Aura virus, Whataroa virus, Highlands J virus, Fort Morgan virus, Buggy Creek virus, Eastern equine encephalitis virus, Middelburg virus, Trocara virus, Kyzylagach virus, Ndumu virus, Babanki virus, Norwegian salmonid alphavirus, Salmon pancreatic disease virus, sleeping disease virus and Southern elephant seal virus; and
more preferably, the alphavirus is selected from the group consisting of Semliki Forest virus, Venezuelan equine encephalitis virus, Sindbis virus and Chikungunya virus.

14. The expression construct according to any one of claims 10 to 13, wherein the 5' cap structure is a natural 5' cap or a 5' cap analog.

15. The expression construct according to any one of claims 1 to 14, comprising a 5' UTR derived from the 5' UTR of any gene or a mutant thereof, preferably a 5' UTR derived from the same virus as which the RNA polymerase derived from.

16. The expression construct according to any one of claims 4 to 15, wherein the replication initiating element is capable of being recognized by the RNA polymerase and guiding the replication of the RNA polymerase; and
preferably, the replication initiating element and the RNA polymerase are derived from the same virus or from different viruses, more preferably from the same virus.

17. The expression construct according to any one of claims 1 to 16, comprising a 3' UTR derived from the 3' UTR of any gene or a mutant thereof, preferably a 3' UTR derived from the same virus as which the RNA polymerase derived from.

18. The expression construct according to any one of claims 1 to 17 comprising a 3' poly(A).

19. The expression construct according to any one of claims 11 to 18, wherein the subgenomic promoter is derived from the promoter of a viral structural protein, and preferably, the subgenomic promoter and the RNA polymerase are derived from the same virus.

20. A vector comprising or encoding the expression construct according to any one of claims 1 to 19.

21. A pharmaceutical composition comprising the expression construct according to any one of claims 1 to 19 or the vector according to claim 20, wherein the target protein is a therapeutic protein.

22. A vaccine composition comprising the expression construct according to any one of claims 1 to 19 or the vector according to claim 20, wherein the target protein is an antigen capable of eliciting a protective immune response, such as an antigen derived from humans, animals, plants, viruses, bacteria and/or parasites.

23. A method for expressing a target protein, comprising introducing the expression construct according to any one of claims 1 to 19 into a target cell, wherein the target cell expresses the target protein from the construct.

24. A method of immunization comprising inoculating a subject in need thereof with the expression construct according to any one of claims 1 to 19.
